(19) Europäisches Patentamt
European Patent Office
Office européen des brevets

(11) EP 2 314 280 A1

(12) # EUROPEAN PATENT APPLICATION
published in accordance with Art. 153(4) EPC

(43) Date of publication:
**27.04.2011 Bulletin 2011/17**

(21) Application number: **09804921.6**

(22) Date of filing: **31.07.2009**

(51) Int Cl.:
***A61K 8/891*** *(2006.01)* ***A61K 8/06*** *(2006.01)*
***A61K 8/40*** *(2006.01)* ***A61K 8/92*** *(2006.01)*
***A61Q 1/10*** *(2006.01)* ***B01F 17/52*** *(2006.01)*
***C11D 1/75*** *(2006.01)* ***C11D 1/88*** *(2006.01)*

(86) International application number:
**PCT/JP2009/063660**

(87) International publication number:
**WO 2010/016437 (11.02.2010 Gazette 2010/06)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **04.08.2008 JP 2008200660**

(71) Applicants:
- **Shiseido Company, Ltd.**
  **Chuo-ku**
  **Tokyo 104-8010 (JP)**
- **Dow Corning Toray Co., Ltd.**
  **Tokyo 100-0005 (JP)**

(72) Inventors:
- **IKEDA Tomoko**
  **Yokohama-shi**
  **Kanagawa 224-8558 (JP)**
- **NISHIHAMA Shuji**
  **Yokohama-shi**
  **Kanagawa 224-8558 (JP)**
- **OSAWA Tomo**
  **Yokohama-shi**
  **Kanagawa 224-8558 (JP)**
- **NAKAMA Yasunari**
  **Yokohama-shi**
  **Kanagawa 224-8558 (JP)**
- **IIMURA Tomohiro**
  **Ichihara-shi**
  **Chiba 299-0108 (JP)**
- **OKAWA Tadashi**
  **Ichihara-shi**
  **Chiba 299-0108 (JP)**

(74) Representative: **Lippert, Stachow & Partner**
**Patentanwälte**
**Frankenforster Strasse 135-137**
**51427 Bergisch Gladbach (DE)**

(54) **COMPLEX, EMULSIFIED COMPOSITION CONTAINING THE SAME, AND WATER-IN-OIL EMULSIFIED COSMETIC MATERIAL**

(57) A stable emulsion cosmetic having an excellent texture in use, and in particular, a solid emulsion cosmetic which is stable in a system containing a large amount of water is provided.

A complex consisting of an organosiloxane derivative represented by the following formula (1) or (2) and an amphoteric surfactant and/or semipolar surfactant.

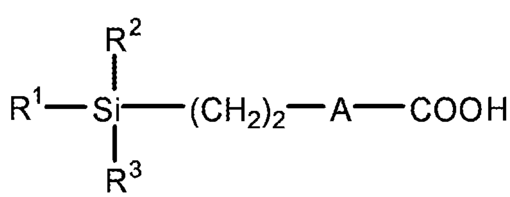

(1)

EP 2 314 280 A1

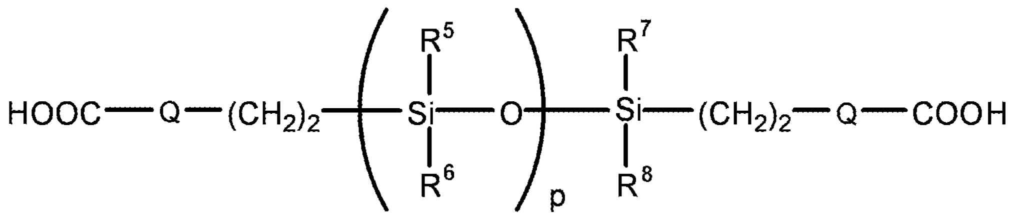 (2)

An emulsion containing the complex, an oil, and water.
A water-in-oil emulsion cosmetic containing the complex, an oil, and water, wherein the system is a water-in-oil type.

## Description

RELATED APPLICATIONS

This application claims the priority of Japanese Patent Application No. 2008-200660 filed on August 4, 2008, which is incorporated herein by reference.

FIELD OF THE INVENTION

The present invention relates to a complex usable as an emulsifier, an emulsion containing the same, and a water-in-oil emulsion cosmetic containing the same, and in particular it relates to the improvement in the technique of solidifying a water-in-oil emulsion containing a large amount of water.

BACKGROUND OF THE INVENTION

Conventionally, solid emulsion cosmetics include a solid water-in-oil emulsion cosmetic in which the outer oil phase is solidified with a specific oily solidifying agent and a solid oil-in-water emulsion cosmetic in which the outer aqueous phase is solidified with a specific aqueous solidifying agent. Among them, because a water-in-oil emulsion cosmetic contains an oil and an oily solidifying agent in its outer phase, it has a problem that it lacks a refreshing feeling and a light fresh feeling compared with an oil-in-water emulsion cosmetic. Therefore, generally, the amount of inner aqueous phase in a water-in-oil emulsion cosmetic has been tried to be increased; however, it was difficult to obtain a stable emulsion system in such a cosmetic. On the other hand, though an oil-in-water emulsion cosmetic can achieve a comparatively excellent feeling in use, it had difficulty in achieving a sufficient hardness, and it also had a problem of being softened over time.

To these problems in solid emulsion cosmetics, it was recently reported that an oil-in-water solid emulsion cosmetic which is excellent in the texture in use could be obtained by incorporating specific amounts of water, a wax ester, and an amphoteric surfactant (see Patent Literature 1). However, as even such an oil-in-water solid emulsion cosmetic was weak in the solidifying power, a sufficient hardness could not be achieved in a system containing a large amount of water, and the stability was also insufficient.

Patent Literature 1: WO96/03107

DISCLOSURE OF THE INVENTION

PROBLEM TO BE SOLVED BY THE INVENTION

The present invention was made in view of the above-described problems, and an object is to provide a stable emulsion cosmetic having an excellent texture in use, and in particular, a solid emulsion cosmetic which is stable in a system containing a large amount of water.

MEANS TO SOLVE THE PROBLEM

The present inventors have diligently studied to solve the above-described problems. As a result, the present inventors have found that, by using a complex, as an emulsifier, consisting of an amphoteric surfactant and/or a semipolar surfactant and a specifically structured organosiloxane derivative with a carboxyl group, a comparatively stable emulsion can be obtained, and a water-in-oil emulsion system containing a large amount of water can be stably solidified. They also found that, with the above-mentioned technique, a solid water-in-oil emulsion cosmetic having a good texture in use and a good stability can be obtained, thus leading to completion of the present invention.

The complex according to the present invention is characterized by consisting of an organosiloxane derivative represented by the following formula (1) or (2) and an amphoteric surfactant and/or a semipolar surfactant.

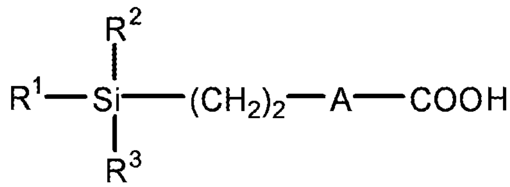

(1)

In the formula (1), at least one of $R^1$ to $R^3$ is a functional group represented by $-O-Si(R^4)_3$ in which $R^4$ is an alkyl group having 1 to 6 carbon atoms or a phenyl group, and the remaining $R^1$ to $R^3$ may be the same or different and each may be a substituted or unsubstituted monovalent hydrocarbon group; A is a linear or branched alkylene group represented by $C_qH_{2q}$ in which q is any integer of 0 to 20; and the organosiloxane derivative represented by the formula (1) contains a total of 2 to 100 silicon atoms (Si) on average per molecule.

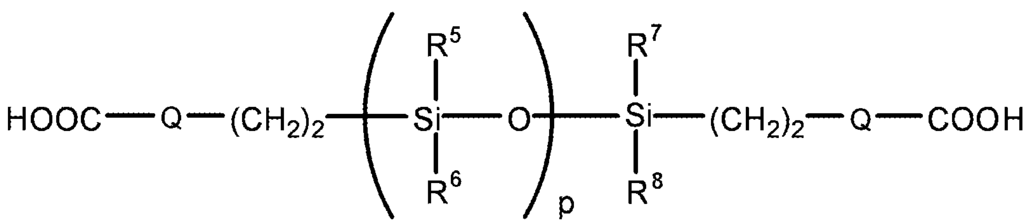

(2)

In the formula (2), $R^5$ to $R^8$ may be the same or different and each is a substituted or unsubstituted monovalent hydrocarbon group; Q is a linear or branched alkylene group represented by $C_qH_{2q}$ in which q is any integer of 0 to 20; and p is any number of 0 to 100.

Moreover, in the complex, it is preferred that the organosiloxane derivative should be represented by the formula (1), wherein $R^1$ and $R^2$ are respectively a functional group represented by $-O-Si(R^4)_3$ in which $R^4$ is an alkyl group having 1 to 6 carbon atoms; $R^3$ is a monovalent hydrocarbon group having 1 to 10 carbon atoms; and q is any integer of 6 to 20.

Moreover, in the complex, it is preferred that the organosiloxane derivative should be represented by the formula (2), wherein $R^5$ to $R^8$ are respectively a group selected from the group consisting of substituted or unsubstituted alkyl groups having 1 to 20 carbon atoms, aryl groups, and aralkyl groups; q is any integer of 6 to 20; and p is any number of 1 to 20.

Moreover, in the complex, it is preferred that the amphoteric surfactant and/or semipolar surfactant should be one or more selected from the compounds represented by the following formulas (A) to (F).

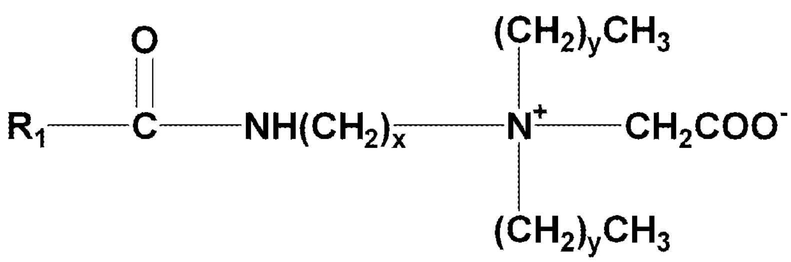

(A)

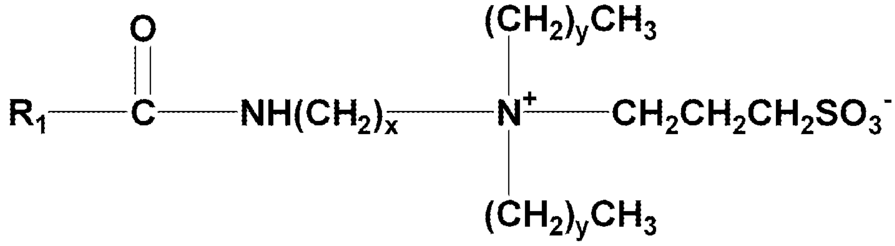

(B)

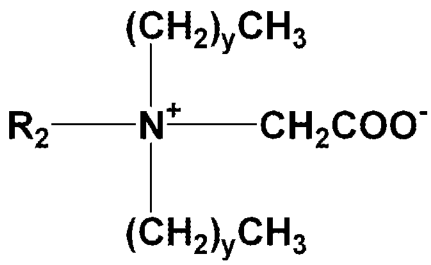

(C)

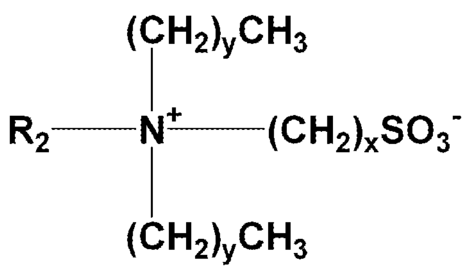

(D)

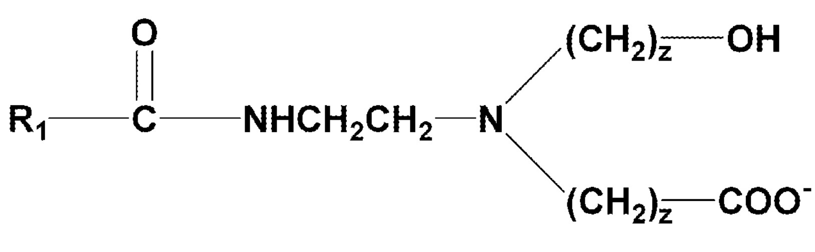

(E)

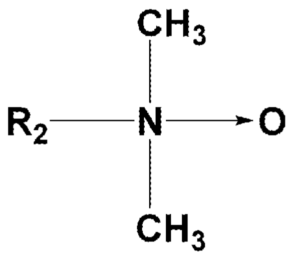

(F)

In the formulas (A) to (F), $R_1$ is an alkyl group or an alkenyl group having 9 to 16 carbon atoms on average; $R_2$ is an alkyl group or an alkenyl group having 10 to 18 carbon atoms on average; x is any integer of 2 to 4; y is any integer of 0 to 4; and z is an integer of 1 or 2.

Moreover, the emulsion according to the present invention is characterized by containing the complex, an oil, and water.

Moreover, the water-in-oil emulsion cosmetic according to the present invention is characterized by containing the complex, an oil, and water.

Moreover, it is preferred that the water-in-oil emulsion cosmetic should further contain a salt and a wax ester and it should be solid.
Moreover, the solid water-in-oil emulsion cosmetic according to the present invention is characterized by containing candelilla wax as the wax ester.

EFFECT OF THE INVENTION

According to the present invention, by using the complex, as an emulsifier, consisting of the amphoteric surfactant and/or semipolar surfactant and the specifically structured organosiloxane derivative with a carboxyl group, the comparatively stable emulsion can be obtained, and particularly a water-in-oil emulsion system containing a large amount of water can be stably solidified. Moreover, with the above-mentioned technique, the solid water-in-oil emulsion cosmetic having a good texture in use and a good stability can be obtained.

BEST MODE FOR CARRYING OUT THE INVENTION

Hereinafter, the preferred embodiments of the present invention will be described.
The complex according to the present invention is characterized by consisting of the specifically structured organosiloxane derivative with a carboxyl group and the amphoteric surfactant and/or semipolar surfactant.

<u>Organosiloxane derivative</u>

The organosiloxane derivative used in the present invention is a compound represented by the formula (1) or (2).
First, the organosiloxane derivative represented by the following formula (1) will be described.

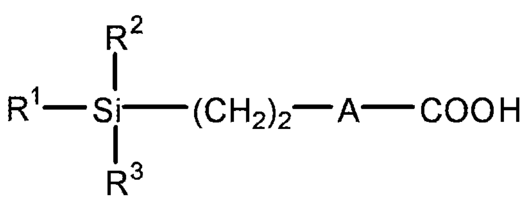

(1)

The organosiloxane derivative represented by the formula (1) is an organosiloxane derivative modified with an alkylcarboxyl group and is characterized by containing a total of 2 to 100 silicon atoms on average per molecule.
In the formula (1), at least one of $R^1$ to $R^3$ is a functional group represented by $-O-Si(R^4)_3$ in which $R^4$ is an alkyl group having 1 to 6 carbon atoms or a phenyl group. In this context, all of $R^1$ to $R^3$ may respectively be any of the functional groups. Alternatively, when at least one of $R^1$ to $R^3$ is any of the functional groups, the remaining $R^1$ to $R^3$ may be the same or different and each may be a substituted or unsubstituted monovalent hydrocarbon group.

In the functional group represented by -O-Si($R^4$)$_3$, $R^4$ is an alkyl group having 1 to 6 carbon atoms or a phenyl group. Examples of the alkyl group having 1 to 6 carbon atoms include linear, branched, or cyclic alkyl groups such as methyl, ethyl, n-propyl, i-propyl, n-butyl, i-butyl, s-butyl, t-butyl, pentyl, neopentyl, cyclopentyl, and hexyl. Examples of the functional group represented by -O-Si($R^4$)$_3$ include -O-Si($CH_3$)$_3$, -O-Si($CH_3$)$_2$($C_2H_5$), -O-Si($CH_3$)$_2$($C_3H_7$), -O-Si($CH_3$)$_2$($C_4H_9$), -O-Si($CH_3$)$_2$($C_5H_{11}$), -O-si($CH_3$)$_2$($C_6H_{13}$), and -O-Si($CH_3$)$_2$($C_6H_5$). In this context, the functional group is preferably a trialkylsiloxy group, most preferably a trimethylsiloxy group.

Moreover, in the formula (1), as long as at least one of $R^1$ to $R^3$ is the functional group represented by -O-Si($R^4$)$_3$, the remaining $R^1$ to $R^3$ may be the same or different and each may be a substituted or unsubstituted monovalent hydrocarbon group. Examples of the unsubstituted monovalent hydrocarbon group as $R^1$ to $R^3$ include: linear, branched, or cyclic alkyl groups such as methyl, ethyl, n-propyl, i-propyl, n-butyl, i-butyl, s-butyl, pentyl, neopentyl, cyclopentyl, and hexyl; aryl groups such as phenyl, tolyl, and xylyl groups; and aralkyl groups. Examples of the substituted monovalent hydrocarbon group as $R^1$ to $R^3$ include: perfluoroalkyl groups such as 3,3,3-trifluoropropyl and 3,3,4,4,4-pentafluorobutyl groups; aminoalkyl groups such as 3-aminopropyl and 3-(aminoethyl)aminopropyl groups; and amidoalkyl groups such as acetylaminoalkyl groups. Moreover, the hydrocarbon group as $R^1$ to $R^3$ may be partially substituted by a hydroxyl, alkoxy, polyether, or perfluoropolyether group. Examples of the alkoxy group include methoxy, ethoxy, and propoxy groups.

In the formula (1), when one or two of $R^1$ to $R^3$ are respectively the functional group represented by -O-Si($R^4$)$_3$, the remaining $R^1$ to $R^3$ are respectively preferably a linear or branched alkyl group having 1 to 6 carbon atoms, particularly preferably a methyl or ethyl group. Particularly, in the formula (1), it is preferred that all or two of $R^1$ to $R^3$ should respectively be the functional group represented by -O-Si($R^4$)$_3$, and the remaining $R^1$ to $R^3$ should be a methyl or ethyl group.

A is a linear or branched alkylene group represented by $C_qH_{2q}$ in which q is any integer of 0 to 20. In this context, when q=0, the organosiloxane derivative represented by the formula (1) is a compound represented by the following formula (1-A), wherein the carboxyl-modified group is bound with silicon via an ethylene group. In the present invention, q is preferably any integer of 2 to 15, more preferably any integer of 6 to 12. On the other hand, if q exceeds the upper limit, it may be difficult to form the complex.

$$R^1R^2R^3Si\text{-}(CH_2)_2\text{-}COOH \qquad (1\text{-}A)$$

Moreover, the organosiloxane derivative represented by the formula (1) is characterized by containing a total of 2 to 100 silicon atoms on average per molecule. The organosiloxane derivative represented by the formula (1) contains preferably a total of 3 to 30 silicon atoms on average. On the other hand, if the total number of the silicon atoms per molecule exceeds 100, it may be difficult to form the complex.

The organosiloxane derivative represented by the formula (1) that can be used preferably is more specifically an organosiloxane derivative wherein $R^1$ and $R^2$ are respectively a functional group represented by -O-Si($R^4$)$_3$ in which $R^4$ is an alkyl group having 1 to 6 carbon atoms; $R^3$ is a linear or branched alkyl group having 1 to 6 carbon atoms; and q is any integer of 6 to 12.

The organosiloxane derivative represented by the formula (1) is obtained by causing addition reaction between polysiloxane containing a silicon-bound hydrogen atom, represented by $R^1R^2R^3SiH$, and a trimethylsilyl carboxylate derivative having a terminal vinyl group, represented by $CH{=}CH_2$-A-$COOSiMe_3$, in the presence of a platinum-based catalyst; adding, to the reaction product, at least 1 mol of a monohydric alcohol (e.g., methanol) per mol of the trimethylsilyl group as a protective group; and heating the mixture to deprotect the protective group by alcoholysis. In this context, $R^1$, $R^2$, $R^3$, and A are as defined above.

Subsequently, the organosiloxane derivative represented by the following formula (2) will be described.

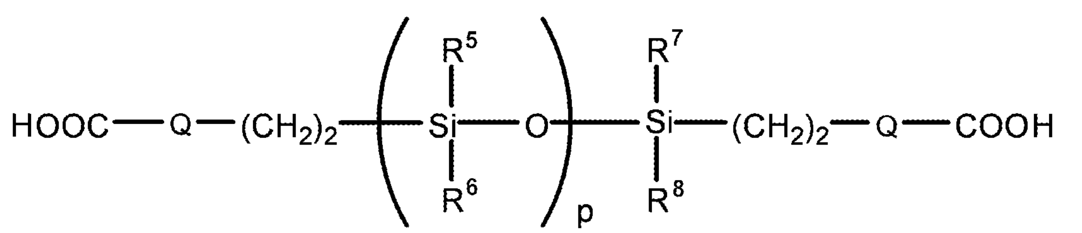

**(2)**

The organosiloxane derivative represented by the formula (2) is an organosiloxane derivative modified, at both ends of the molecular chain, with an alkylcarboxyl group.

In the formula (2), $R^5$ to $R^8$ may be the same or different and are selected from substituted or unsubstituted monovalent hydrocarbon groups. Examples of the unsubstituted monovalent hydrocarbon group as $R^5$ to $R^8$ include: linear or

branched alkyl groups such as methyl, ethyl, propyl, butyl, pentyl, hexyl, heptyl, octyl, decyl, and dodecyl groups; linear or branched alkenyl groups such as allyl and hexenyl groups; cycloalkyl groups such as cyclopentyl and cyclohexyl groups; aryl groups such as phenyl, tolyl, and naphthyl groups; and aralkyl groups such as benzyl, phenylethyl, phenylpropyl, naphthylmethyl, and naphthylethyl groups. Examples of the substituted monovalent hydrocarbon group as $R^5$ to $R^8$ include the groups in which hydrogen atoms bound with the carbon atoms of the hydrocarbon groups described above are partially substituted by an organic group such as a hydroxyl group, a halogen atom, an epoxy group, an amino group, a methacryl group, a mercapto group, an alkoxy group, a polyether group, or a perfluoropolyether group, and specifically include: perfluoroalkyl groups such as 3,3,3-trifluoropropyl and 3,3,4,4,4-pentafluorobutyl groups; aminoalkyl groups such as 3-aminopropyl and 3-(aminoethyl)aminopropyl groups; and amidoalkyl groups such as acetylaminoalkyl groups. $R^5$ to $R^8$ are respectively preferably an alkyl group having 1 to 20 carbon atoms, an aryl group, or an aralkyl group. It is particularly preferred that 90% by mol or more of $R^5$ to $R^8$ in one molecule should be a methyl group and/or a phenyl group.

Q is a linear or branched alkylene group represented by $C_qH_{2q}$, in which q is any integer of 0 to 20. In this context, when q=0, the organosiloxane derivative represented by the formula (2) is a compound represented by the following formula (2-A), wherein the carboxyl-modified group is bound with silicon via an ethylene group. In the present invention, q is preferably any integer of 6 to 20, particularly preferably any integer of 6 to 12. On the other hand, if q exceeds the upper limit, it may be difficult to form the complex.

$$HOOC\text{-}(CH_2)_2\text{-}(SiR^5R^6\text{-}O)_p\text{-}SiR^7R^8\text{-}(CH_2)_2\text{-}COOH \qquad (2\text{-}A)$$

In the above-mentioned formula, $R^5$ to $R^8$ are as defined above, and p is any number of 0 to 100.

In the formula (2), p specifies the average degree of polymerization of di-substituted polysiloxane and is any number of 0 to 100. In the present invention, p is more preferably any number of 1 to 20, particularly preferably any number of 1 to 10. On the other hand, if p exceeds the upper limit, it may be difficult to form the complex.

The organosiloxane derivative represented by the formula (2) that can be used preferably is an organosiloxane derivative wherein $R^5$ to $R^8$ are respectively an alkyl group having 1 to 6 carbon atoms; q is any integer of 0 to 20; and p is any number of 0 to 20.

The organosiloxane derivative represented by the formula (2) is obtained by causing addition reaction between organohydrogenpolysiloxane having a silicon-bound hydrogen atom at both ends of the molecular chain, represented by

$$H\text{-}(SiR^5R^6\text{-}O)_p\text{-}SiR^7R^8\text{-}H$$

(wherein $R^5$ to $R^8$, p, and q are as defined above),
and at least 2 mol of a trimethylsilyl carboxylate derivative having a terminal vinyl group, represented by $CH{=}CH_2$-Q-$COOSiMe_3$, with respect to 1 mol of the organohydrogenpolysiloxane in the presence of a platinum-based catalyst; adding, to the reaction product, at least 1 mol or more of a monohydric alcohol (e.g., methanol), water, or the mixture thereof per mol of the trimethylsilyl group as a protective group; and heating the mixture to deprotect the protective group by alcoholysis. In this context, Q is as defined above.

The platinum-based catalyst used for producing the organosiloxane derivative of the present invention represented by the formula (1) or (2) is a catalyst for hydrosilylation reaction between the silicon-bound hydrogen atom and the alkenyl group. Examples thereof include chloroplatinic acid, alcohol-modified chloroplatinic acid, olefin complexes of platinum, ketone complexes of platinum, vinylsiloxane complexes of platinum, platinum tetrachloride, fine platinum powder, solid platinum supported by an alumina or silica carrier, platinum black, olefin complexes of platinum, alkenylsiloxane complexes of platinum, carbonyl complexes of platinum, and thermoplastic organic resin (e.g., methyl methacrylate, polycarbonate, polystyrene, and silicone resins) powders containing these platinum-based catalysts. The platinum-based catalyst is preferably a 1,3-divinyl-1,1,3,3-tetramethyldisiloxane complex of platinum or chloroplatinic acid.

Amphoteric surfactant/Semipolar surfactant

The amphoteric surfactant and semipolar surfactant used in the present invention are not limited in particular as long as they are amphoteric surfactants or semipolar surfactants used in general cosmetic bases and so on.

Examples of the amphoteric surfactant usable in the present invention will be described in the following.
Amide betaine type amphoteric surfactants represented by the following formula (A) (e.g., commercially available products such as Lebon 2000 manufactured by Sanyo Chemical Industries, LTD. and Anon BDF manufactured by NOF Corporation).

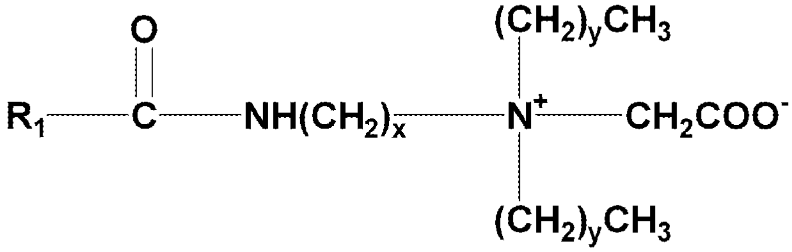

(A)

Amide sulfobetaine type amphoteric surfactants represented by the following formula (B) (e.g., commercially available products such as Lonzaine-CS manufactured by Lonza andIrataine CBS manufactured by Miranol).

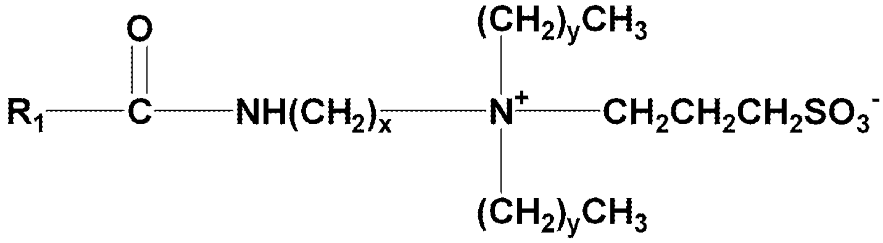

(B)

Betaine type amphoteric surfactants represented by the following formula (C) (e.g., commercially available products such as Anon BL manufactured by NOF Corporation and Dehyton AB-30 manufactured by Henkel KGaA.).

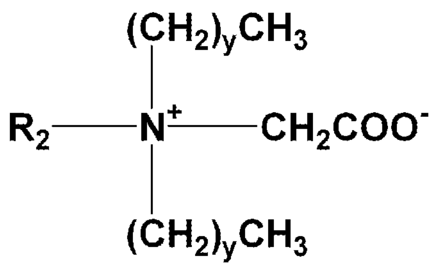

(C)

Sulfobetaine type amphoteric surfactants represented by the following formula (D) (e.g., commercially available products such as Lonzaine 12CS manufactured by Lonza).

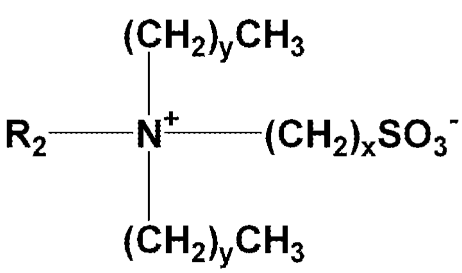

(D)

Imidazolinium type amphoteric surfactants represented by the following formula (E) (e.g., commercially available products such as Obazoline 662-N manufactured by Toho Chemical Industry Co., Ltd. and Anon GLM manufactured by NOF Corporation).

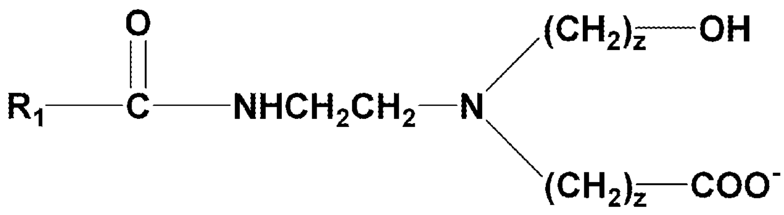

(E)

Specific examples of the semipolar surfactant usable in the present invention include tertiary amine oxide type semipolar surfactants represented by the following formula (F) (e.g., commercially available products such as Unisafe A-LM manufactured by NOF Corporation and Wondamine OX-100 manufactured by New Japan Chemical Co., Ltd.).

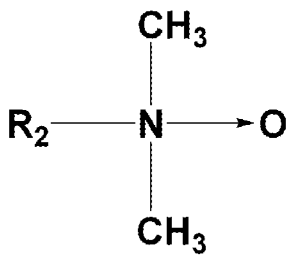

(F)

In the formulas (A) to (F), $R_1$ is preferably an alkyl group or an alkenyl group having 9 to 21 carbon atoms on average, more preferably an alkyl group or an alkenyl group having 11 to 17 carbon atoms on average, and most preferably an alkyl group or an alkenyl group having 11 to 13 carbon atoms on average. When the average number of carbon atoms is less than 9, the hydrophilicity is so strong that it is difficult to form the complex. When it exceeds 21, the water solubility becomes so poor that it is difficult to form the complex.
$R_2$ represents an alkyl group or an alkenyl group having 10 to 18 carbon atoms on average. x is any integer of 2 to 4, y is any integer of 0 to 3, and z is an integer of 1 or 2.
In the present invention, any one of these amphoteric surfactants and semipolar surfactants can be used alone, or two or more of them can be used in combination.

<u>Complex</u>

The complex according to the present invention is characterized by consisting of the amphoteric surfactant and/or semipolar surfactant and the specifically structured organosiloxane derivative with a carboxyl group.
The specifically structured organosiloxane derivative is added to and mixed with the amphoteric surfactant and/or semipolar surfactant solution, resulting in the formation of the complex which is insoluble in both of water and oil. The complex has a chemical composition which is different from that of the organosiloxane derivative or the amphoteric surfactant and/or semipolar surfactant, and it is not a mixture of the organosiloxane derivative and the amphoteric surfactant and/or semipolar surfactant. The complex can be thought to be bonded, at the carboxyl group portion of the organosiloxane derivative, to the amphoteric surfactant and/or semipolar surfactant.
In the complex according to the present invention, it is desirable that the blending ratio, in mass ratio, of the organosiloxane derivative and the amphoteric surfactant and/or semipolar surfactant should be preferably 0.5:9.5 to 9.5:0.5 [(organosiloxane derivative)/(amphoteric surfactant and/or semipolar surfactant) = 0.05 to 19], and more preferably 1:9 to 9:1 [(organosiloxane derivative)/(amphoteric surfactant and/or semipolar surfactant) = 0.1 to 9]. When the complex is used as an emulsifier, the blending ratio can be suitably set depending upon a desired emulsion type. By setting the blending ratio to 0.5:9.5 to 9.5:0.5 or 1:9 to 9:1, the stability of emulsion is further improved when the complex is used as an emulsifier.
The complex according to the present invention can be produced, for example, as follows.
The amphoteric surfactant and/or semipolar surfactant is mixed with water to prepare a surfactant solution. To this amphoteric surfactant and/or semipolar surfactant solution, the organosiloxane derivative is added. In this context, as necessary, this addition is preferably performed while the mixture is stirred with, for example, a stirrer. When the organosiloxane derivative is added to the amphoteric surfactant and/or semipolar surfactant solution, the amphoteric surfactant and/or semipolar surfactant adsorbs to the organosiloxane derivative and bonds to the carboxyl group portion of the organosiloxane derivative. The adsorption of the amphoteric surfactant and/or semipolar surfactant to the organosiloxane derivative increases as time advances, and becomes saturated after the elapse of a certain period of time.

The complex which became solid and suspended can be easily separated and colleted by centrifuging the solution obtained after the addition.

The complex according to the present invention can be preferably used, for example, as an emulsifier. Under the coexistence of water and oil, as the complex is aligned at the interface between water and oil and functions as a strong interface film at the emulsion particle interface, the coalescence of emulsion particles can be prevented from occurring. The complex also functions as an emulsifier having a wide range of emulsifying ability which is not subject to the variation in the required HLB of oil. Furthermore, as the HLB can be adjusted by changing the blending ratio of the organosiloxane derivative and the amphoteric surfactant and/or semipolar surfactant, the emulsion type can be easily selected.

## Emulsion

The emulsion according to the present invention can be obtained by using the complex thus obtained as an emulsifier. The emulsion can be prepared by adding the complex thus obtained to a solution containing water and oil, if necessary, under stirring and/or heating. In a normal method, it takes 2 to 3 hours to prepare a stable emulsion system. However, in the present invention, it takes only about 1 hour by using the complex, and furthermore, the conventional process for producing an emulsion can be simplified and curtailed, for example; the emulsion can be completely emulsified by propeller stirring.

In terms of the ease of preparation, the emulsion also can be prepared in the following method. The organosiloxane derivative is added to the oil, and the mixture is stirred with a disperser at a normal temperature if it is liquid at the normal temperature or in a melted state by heating if it is solid at the normal temperature. Then, while the stirring is continued, the amphoteric surfactant and/or semipolar surfactant solution is gradually added to the mixture to prepare the emulsion. When it is difficult to dissolve the organosiloxane derivative in the oil, the emulsification efficiency can be improved by adding a solvent, such as isoparaffin, to the mixture. The additive components other than the emulsifier are added to the emulsion promptly after the above-mentioned preparation steps, and then stirred lightly.

The oil used in the emulsion of the present invention are not limited in particular, and the examples include liquid oils such as avocado oil, camellia oil, turtle oil, macadamia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, Japanese tung oil, jojoba oil, germ oil, triglycerin, glyceryl trioctanoate, pentaerythritol tetraoctanoate, and glyceryl triisopalmitate; solid oils such as cacao butter, coconut oil, hardened coconut oil, palm oil, palm kernel oil, Japan wax kernel oil, and hardened oil; hydrocarbon oils such as liquid paraffin, ozokerite, squalene, pristane, paraffin, and squalane; fatty acids such as stearic acid, lauric acid, myristic acid, behenic acid, isostearic acid, oleic acid, and 12-hydroxystearic acid; higher alcohols such as stearyl alcohol, cetyl alcohol, lauryl alcohol, oleyl alcohol, isostearyl alcohol, and behenyl alcohol; fluorinated oils such as perfluorodecane, perfluorooctane, and perfluoropolyether; cyclic silicones such as decamethylcyclopentasiloxane and octamethylcyclotetrasiloxane; and silicone oils such as dimethylpolysiloxane, methylphenylpolysiloxane, alkoxy modified silicone, and alkyl modified silicone. One of the above-described oils can be used alone, or two or more of them can be used in combination.

In the total amount of the emulsion, the sum amount of the organosiloxane derivative and the amphoteric surfactant and/or semipolar surfactant is preferably 0.1 to 30 % by mass, and more preferably 0.5 to 20 % by mass. When the sum amount is 0.1 % by mass or more or 0.5 % by mass or more, the stability of the emulsion can be improved. As the effect becomes saturated when the sum amount is more than 30 % by mass, the upper limit is preferably 30 % by mass in economic terms. Also, in the total amount of the emulsion, it is preferred that the amount of water should be 10 to 80 % by mass and the amount of oil should be 10 to 80 % by mass. When an O/W emulsion is prepared, it is preferred that the amount of water should be 10 to 50 % by mass and the amount of oil should be 30 to 70 % by mass in the total amount of the emulsion.

Though the emulsion type of the emulsion obtained in the above-mentioned method varies depending on the kind of oil, the ratio of oil and water, and so on, the emulsion type varies around the mass ratio of 1 to 2 in the organosiloxane derivative/the amphoteric surfactant and/or semipolar surfactant. Generally, an O/W emulsion tends to be obtained when the mass ratio is 1 to 2 or less, and a W/O emulsion tends to be obtained when the mass ratio is 1 to 2 or more. By using a preparation method such as a phase-transfer emulsification, it becomes possible to obtain a stable multiphase-type emulsion such as W/O/W or O/W/O type.

## Water-in-oil emulsion cosmetic

The water-in-oil emulsion cosmetic according to the present invention can be prepared by using the complex as an emulsifier. By using the complex, the stable water-in-oil emulsion cosmetic having an excellent texture in use can be obtained.

In the water-in-oil emulsion cosmetic according to the present invention, it is more preferred that, in the total amount of

the cosmetic, the amount of water should be 30 to 70 % by mass and the amount of oil should be 10 to 50 % by mass. As mentioned above, the stable water-in-oil emulsion system containing a large amount of water can be obtained by using the complex, and thus it becomes possible to obtain the water-in-oil emulsion cosmetic having an excellent texture in use as well as a good stability.

It is preferred that the water-in-oil emulsion cosmetic according to the present invention should further contain a salt and a wax ester. By incorporating the salt and the wax ester together with the essential components, the solid water-in-oil emulsion cosmetic in which the water-in-oil emulsion system containing a large amount of water is stably solidified can be obtained.

In the present invention, the term "solid" means being in a state that a composition does not show the fluidity at the temperature of 50 ˚C or less. More specifically, for example, it means being in a state that the hardness γ of a composition, measured with a known measurement device such as a rheometer (manufactured by Fudo Industries Co., Ltd.) and represented by the following formula, is 30 or more.

$$\gamma = (G^*L)/(l^*a) \qquad (dyn/cm^2)$$

(wherein, G: Measuring stress (gr) * 980 dyn, L: Thickness of sample (mm), 1: Compression distance (mm), a: Cross-sectional area of penetrator ($cm^2$))

(Measurement condition)

Load weight: 2 kg, Diameter of penetrator: 5.6 φ, Penetration speed: 2 cm/min, Penetration distance: 1 mm, Measurement temperature: 37˚C

In the solid water-in-oil emulsion cosmetic of the present invention, the amount of salt is preferably 0.1 to 3.0 % by mass in the total amount of the cosmetic, and more preferably 0.2 to 2.0 % by mass. The amount of wax ester is preferably 1.0 to 15.0 % by mass in the total amount of the cosmetic, and more preferably 2.0 to 10.0 % by mass. When the amounts of salt and wax deviate from the ranges, the emulsion system may become unstable or may not be solidified.

Examples of salt usable in the solid water-in-oil emulsion cosmetic of the present invention include sodium chloride, potassium chloride, magnesium chloride, magnesium sulfate, L-sodium glutamate, L-sodium aspartate, sodium pyrrolidonecarboxylate, sodium lactate, sodium citrate, sodium malate, and sodium tartrate.

The wax ester used in the solid water-in-oil emulsion cosmetic of the present invention is solid or semisolid at a normal temperature and contains higher fatty acid esters. Generally, it contains a carboxylic acid ester composed of a portion derived from a higher fatty acid having 18 to 34 carbon atoms and a portion derived from a higher fatty alcohol having 18 to 44 carbon atoms. The portions derived from the fatty acid and fatty alcohol can be liner or branched and be saturated or unsaturated; however, a saturated aliphatic group is preferred. Though A natural wax generally contains free fatty acids, free alcohols, and/or hydrocarbons, in addition to the fatty acid esters, the wax ester used in the present invention can be a wax containing these components.

Specific examples of wax esters usable in the solid water-in-oil emulsion cosmetic of the present invention include beeswax, candelilla wax, cotton wax, carnauba wax, bayberry wax, Chinese wax, spermaceti wax, montan wax, rice bran wax, lanolin, kapok wax, Japan wax, lanolin acetate, liquid lanolin, sugar cane wax, isopropyl lanolate, hexyl laurate, reduced lanolin, jojoba wax, hard lanolin, shellac wax, beeswax, microcrystalline wax, paraffin wax, POE lanoline alcohol ether, POE lanoline alcohol acetate, POE cholesterol ether, lanolin fatty acid polyethylene glycol, fatty acid glyceride, hardened castor oil, petrolatum, and POE hydrogenated lanolin alcohol ether. These wax esters can be used in combination with each other, and they also can be used in combination with other solid or liquid oils.

In the solid water-in-oil emulsion cosmetic of the present invention, it is particularly preferred that a candelilla wax should be used as the wax ester. By selectively incorporating a candelilla wax in particular, it is possible to obtain the solid emulsion cosmetic which is stable particularly in a water-in-oil emulsion system containing a large amount of water. In this context, other wax esters can be incorporated in combination.

The water-in-oil emulsion cosmetic according to the present invention can be suitably prepared according to the above-mentioned method for producing the emulsion. Specifically, for example, the organosiloxane derivative is gradually added to the oil, the mixture is stirred under heating as necessary, and a solution of amphoteric surfactant and/or semipolar surfactant is gradually added to the mixture while the stirring is continued to prepare the water-in-oil emulsion cosmetic. Also, after the preparation of the water-in-oil emulsion cosmetic, other components including the salt and wax are added thereto and mixed therewith as necessary to obtain the solid water-in-oil emulsion cosmetic. In this context, the other components including the salt and wax can be added, in advance, to the organosiloxane derivative oily solution or the amphoteric surfactant and/or semipolar surfactant solution as long as they don't deteriorate the formation of the complex.

In the water-in-oil emulsion cosmetic according to the present invention, a powder such as a pigment, a resin powder, and a spherical powder can be incorporated in addition to the above-mentioned essential components. The powder used is not limited in particular as long as it is generally used in cosmetics. Examples of inorganic pigments include talc, kaolin, calcium carbonate, zinc oxide, titanium dioxide, low-order titanium oxide, red iron oxide, yellow iron oxide, black iron oxide, ultramarine, mangoviolet, cobaltviolet, pearlescent pigments such as titanium oxide coated mica, titanium oxide coated bismuth oxychloride, titanium oxide coated talc, colored titanium oxide coated mica, bismuth oxychloride, and fish scale flake, iron oxide (red iron oxide), binding pigment, ultramarine pink, chromium hydroxide, titanated mica, chromium oxide, cobalt aluminum oxide, ultramarines, Prussian blue, carbon black, copper powder, silicic anhydride, magnesium silicate, bentonite, mica, zirconium oxide, magnesium oxide, zinc oxide, titanium oxide, light calcium carbonate, heavy calcium carbonate, light magnesium carbonate, heavy magnesium carbonate, calamine, Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404, and zirconium lakes, barium lakes, and aluminum lakes, such as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1.

Examples of spherical powder include spherical powders composed of organic polymers such as polystyrene, nylon, acrylic polymer, styrene, polypropylene, polyurethane, cellulose, and silicone (e.g., organopolysiloxane elastomer), and copolymers thereof.

Depending on the product form, the powder is preferably hydrophobized. Examples of the hydrophobized powder include a powder surface-treated by a high-viscosity silicone, a powder surface-coated with a silicone resin with which an alkyl hydrogen polysiloxane is reacted, a powder that the silicone resin-coated powder is further alkene-treated, a powder treated by one or more of a cationic surfactant, an anionic surfactant, and a nonionic surfactant, and a powder surface-coated with a wax; however, the method is not limited in particular so far as the surface of the powder becomes hydrophobized. The amount of these powders is preferably 0.1 to 50 % by mass in the total amount of the cosmetic.

In the water-in-oil emulsion cosmetic according to the present invention, other components generally used in cosmetics, pharmaceuticals, and so on can be arbitrarily incorporated, as necessary, within the range that the effect of the present invention is not undermined. Examples thereof include UV protection agents, anionic surfactants, cationic surfactants, nonionic surfactants, moisturizers, water-soluble polymers, thickeners, coating agents, metal ion sequestering agents, lower alcohols, polyhydric alcohols, pH adjusters, antioxidants, antioxidant aids, and perfumes.

The use application of the water-in-oil emulsion cosmetic according to the present invention is not limited in particular, and the cosmetic can be applied in products such as pre-makeup, foundation, cheek color, eye shadow, eye liner, sunscreen products, and body-care cosmetics.

EXAMPLES

Hereinafter, the present invention will be described more specifically with reference to examples; however, the present invention is not limited thereto.

The structures and synthesis methods of the organosiloxane derivatives used in the examples and comparative examples (Compounds 1 to 4 and Comparative Compounds 1 to 4) are shown below. In this context, each compound was identified by $^{1}H$,$^{13}C$,$^{29}Si$-NMR (NMR apparatus: Fourier Transform Nuclear Magnetic Resonance Spectrometer JEOL JNM-EX400 (manufactured by JEOL Ltd.)).

Compound 1

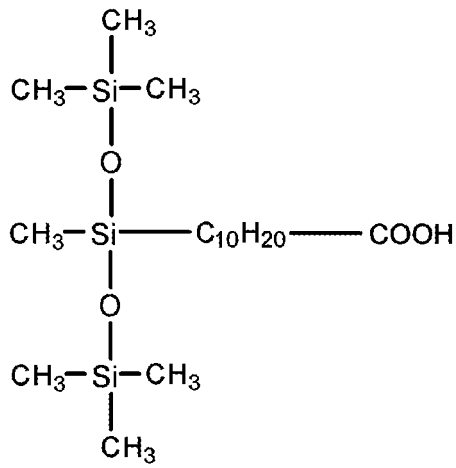

Synthesis method of Compound 1

100 g of 1,1,1,3,5,5,5-heptamethyltrisiloxane and 0.02 g of a toluene solution of a platinum-1,3-divinyl-1,1,3,3-tetramethyldisiloxane complex were added to a flask equipped with a stirrer, a reflux condenser, a dropping funnel, and a thermometer. While the temperature was kept in the range of 70 to 100˚C, 105 g of trimethylsilyl undecylenate was added dropwise to the flask. After the completion of the dropwise addition, the mixture was aged at 100˚C for 2 hours, and the completion of the reaction was then confirmed using gas chromatography. Low-boiling fractions were distilled off under reduced pressure. Then, methanol and water were added thereto, and the mixture was aged for 5 hours under reflux for deprotection. Then, low-boiling fractions were removed again under reduced pressure to obtain Compound 1. As a result of analysis, Compound 1 was confirmed to be represented by the chemical structural formula shown above.

Compound 2

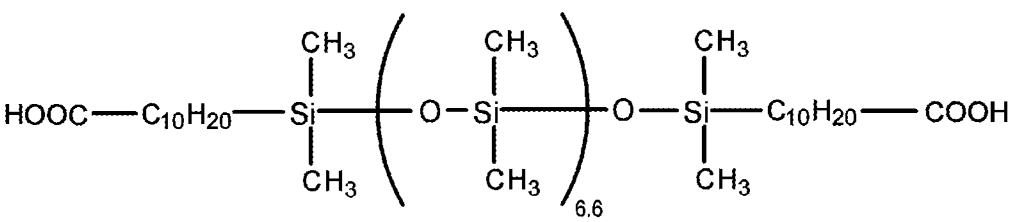

Synthesis method of Compound 2

225.0 g of trimethylsilyl undecylenate and 0.05 g of a toluene solution of a platinum-1,3-divinyl-1,1,3,3-tetramethyldisiloxane complex were added to a flask equipped with a stirrer, a reflux condenser, a dropping funnel, and a thermometer. While the temperature was kept in the range of 70 to 80˚C, 225 g of siloxane having Si-H at both ends, represented by the following formula, was added dropwise to the flask.

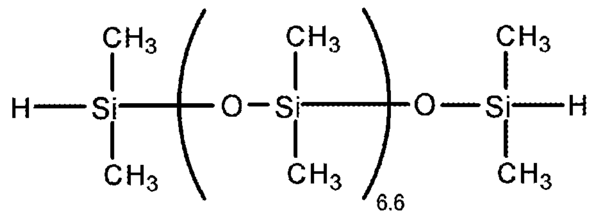

After the completion of the dropwise addition, the mixture was aged at 100˚C for 2 hours, and the disappearance of the Si-H bonds was then confirmed by FT-IR. Low-boiling fractions were distilled off under reduced pressure. Then, 127 g of methanol was added thereto, and the mixture was aged for 5 hours under reflux for deprotection. Then, low-boiling fractions were removed again under reduced pressure to obtain Compound 2. As a result of analysis, Compound 2 was confirmed to be represented by the chemical structural formula shown above.

The present inventors tried preparing the emulsion by using the complex of each of the organosiloxane derivatives thus prepared and the amphoteric surfactant, and studied the emulsification capacity. The compositions used in the test and the results are together shown in Table 1. In this context, the method for producing the compositions and the details of evaluation are as shown below.

<Method for producing emulsion>

While the amphoteric surfactant dissolved in an ion-exchanged water was stirred with a homogenizer, each of the organosiloxane derivatives dissolved in the oil was added thereto and mixed therewith to prepare the emulsion.

<Stability of emulsion>

The obtained composition was allowed to stand at a normal temperature for 1 month. Then, using a microscope, the emulsion particle size of the composition was compared with that of a composition just after the preparation, and the stability was evaluated in accordance with the following criteria.
O: The emulsion particles didn't coalesce at all.
Δ: The emulsion particles coalesced slightly.
X: The emulsion particles coalesced significantly, and the separation was caused.

<Emulsion type>

By using a conductivity method and observing with a microscope, the judgment on whether the emulsion type of the obtained composition was O/W (oil-in-water) or W/O (water-in-oil) was made.

Table 1

| | | Test Ex. 1-1 | Test Ex 1-2 | Test Ex. 1-3 | Test Ex. 1-4 | Test Ex. 1-5 | Test Ex. 1-6 |
|---|---|---|---|---|---|---|---|
| Oil | Decamethylcyclopentasiloxane | 38.5 | 38.5 | 56.5 | 56.5 | 38.5 | 40.0 |
| Emulsifier | Compound1 | 1.5 | - | 3.5 | - | 1.5 | - |
| | Compound2 | - | 1.5 | - | 3.5 | - | - |
| | Sodium 2-undecyl-N,N,N-(hydroxyethylcarboxymethyl)-2-imidazolate (Real content 30%)* | 4.0 | 4.0 | 4.0 | 4.0 | - | 4.0 |
| Ion exchanged water | | Balance | Balance | Balance | Balance | Balance | Balance |
| Emulsion type | | O/W | O/W | W/O | W/O | - | W/O O/W |
| Stability of emulsion | | O | O | O | O | × | Δ |

* Obazoline 662N (manufactured by Toho Chemical Industry Co., Ltd.)

As shown in Table 1, each of Test Examples 1-1 to 1-4, emulsified with use of the organosiloxane derivative of Compound 1 or 2 and the amphoteric surfactant (sodium 2-undecyl-N,N,N-(hydroxyethylcarboxymethyl)-2-imidazolate), was the stable emulsion having the emulsion type different depending on the blending ratio of oil/water and the mass ratio of organosiloxane derivative/amphoteric surfactant. On the other hand, Test Example 1-5, in which the organosiloxane derivative was used alone, could not be emulsified, and Test Example 1-6, in which the amphoteric surfactant was used alone, was poor in the stability of emulsion.

Moreover, the present inventors prepared the water-in-oil emulsion cosmetic containing a large amount of water by using the complex of the organosiloxane derivative and the amphoteric surfactant, and studied the solidified state. The compositions used in the test and the results are together shown in Table 2. In this context, the details for evaluating the solidified state are as shown below.

<Solidification of system>

For each of the obtained compositions, the hardness γ represented by the following formula was measured with a rheometer (manufactured by Fudo Industries Co., Ltd.), and the solidified state of the system was evaluated in accordance with the following criteria.

$$\gamma = (G*L)/(l*a) \qquad (dyn/cm^2)$$

(wherein, G: Measuring stress (gr) * 980 dyn, L: Thickness of sample (mm), 1: Compression distance (mm), a: Cross-sectional area of penetrator ($cm^2$))

(Measurement condition)

Load weight: 2 kg, Diameter of penetrator: 5.6 φ, Penetration speed: 2 cm/min, Penetration distance: 1 mm, Measurement temperature: 37˚C

(Criteria)

⊙: The hardness of composition was 50 or more.
O: The hardness of composition was 30 or more and less than 50.
X: The hardness of composition was less than 30.

Table 2

| | | Test Ex. 2-1 | Test Ex. 2-2 | Test Ex. 2-3 | Test Ex. 2-4 | Test Ex. 2-5 | Test Ex. 2-6 | Test Ex. 2-7 |
|---|---|---|---|---|---|---|---|---|
| Oil | Decamethylcyclopentasiloxane | 35.5 | 35.5 | 35.5 | 35.5 | 35.5 | 35.5 | 35.5 |
| Emulsifier | Compound1 | 1.5 | 1.5 | 1.5 | 1.5 | 1.5 | 1.5 | 1.5 |
| | Sodium 2-undecyl-N,N, N-(hydroxyethylcarboxymethyl)-2-imidazolate (Real content 30%)* | 4.0 | 4.0 | 4.0 | 4.0 | 4.0 | 4.0 | 4.0 |
| Ion exchanged water | | Balance | Balance | Balance | Balance | Balance | Balance | Balance |
| Salt | NaCl | - | 0.2 | 0.4 | 0.8 | 1.2 | - | 1.6 |
| Wax | Candelilla wax | 3.0 | 3.0 | 3.0 | 3.0 | 3.0 | - | - |
| | Rice wax | - | - | - | - | - | 3.0 | 3.0 |
| Emulsion type | | W/O =>O/W | W/O | W/O | W/O | W/O | W/O =>O/W | W/O (O/W) |
| Solidification of system | | × | ○ | ○ | ○ | ○ | × | ○ |

* Obazoline 662N (manufactured by Toho Chemical Industry Co., Ltd.)

As shown in Table 2, each of Test Examples 2-2 to 2-5 and 2-7, in which the salt and the wax ester is incorporated together with the complex of the organosiloxane derivative/amphoteric surfactant, was the emulsion wherein the system was sufficiently solidified in spite of being a water-in-oil emulsion system containing a relatively large amount of water. On the other hand, each of Test Examples 2-1 to 2-6, in which the salt was not incorporated, had the inversion from the water-in-oil phase to the oil-in-water phase, and could not achieve a sufficient hardness. In this context, though Test Example 2-7, in which a rice wax was used, was a solid water-in-oil emulsion, the emulsion system was very unstable. Thus, as the hardness may be lowered owing to the phase inversion to an oil-in-water phase, it can be thought that a candelilla wax is more preferably used.

Hereinafter, specific formulation examples of the solid water-in-oil emulsion cosmetic of the present invention in which the organosiloxane derivative, the amphoteric surfactant, the salt, and the wax ester are incorporated will be shown; however, the present invention is not limited thereto.

| Formulation example 1: W/O solid emulsion foundation | (mass %) |
|---|---|
| (1) Decamethylcyclopentasiloxane | Balance |
| (2) Methylpolysiloxane | 6.0 |
| (3) 2-Ethylhexyl p-methoxycinnamate | 3.0 |
| (4) Compound 1 | 3.0 |
| (5) Candelilla wax | 4.0 |
| (6) Silicone treated fine titanium oxide | 10.0 |
| (7) Alkyl-modified silicone resin-coated yellow iron oxide | 1.8 |
| (8) Alkyl-modified silicone resin-coated red iron oxide | 0.5 |
| (9) Alkyl-modified silicone resin-coated black iron oxide | 0.15 |
| (10) Alkyl-modified silicone resin-coated sericite | 3.55 |
| (11) 1,3-Butylene glycol | 5.0 |
| (12) Methyl paraben | 0.15 |
| (13) Ion-exchanged water | 50.0 |
| (14) Sodium 2-undecyl-N,N,N-(hydroxyethylcarboxymethyl) -2-imidazolate (Real content: 30%) | 4.0 |
| (15) Sodium chloride | 1.0 |

(Production method) (1) to (5) were heated to 80 ˚C, and (6) to (10) were mixed therein. To the obtained oil phase solution, an aqueous phase solution in which (11) to (15) were mixed and heated to 80 ˚C was gradually added. The mixture was stirred with a homomixer and cooled to obtain the W/O emulsion foundation of Formulation example 1.

| Formulation example 2: W/O solid emulsion pre-makeup | (mass %) |
|---|---|
| (1) Decamethylcyclopentasiloxane | Balance |
| (2) Methylpolysiloxane | 2.0 |
| (3) 2-Ethylhexyl p-methoxycinnamate | 5.0 |
| (4) Compound 1 | 3.0 |
| (5) Candelilla wax | 4.0 |
| (6) Silicone treated fine titanium oxide | 2.0 |
| (7) Alkyl-modified silicone resin-coated yellow iron oxide | 0.2 |
| (8) Alkyl-modified silicone resin-coated red iron oxide | 0.05 |
| (9) Polymethyl methacrylate | 8.0 |
| (10) Alkyl-modified silicone resin-coated sericite | 3.75 |
| (11) 1,3-Butylene glycol | 5.0 |
| (12) Methyl paraben | 0.15 |
| (13) Ion-exchanged water | 50.0 |
| (14) Sodium 2-undecyl-N,N,N-(hydroxyethylcarboxymethyl) -2-imidazolate (Real content: 30%) | 4.0 |
| (15) Sodium chloride | 1.0 |

(Production method) (1) to (5) were heated to 80˚C, and (6) to (10) were mixed therein. To the obtained oil phase solution, an aqueous phase solution in which (11) to (15) were mixed and heated to 80 ˚C was gradually added. The mixture was stirred with a homomixer and cooled to obtain the W/O emulsion pre-makeup of Formulation example 2.

| Formulation example 3: W/O solid emulsion mascara | (mass %) |
|---|---|
| (1) Isoparaffin | Balance |
| (2) Trimethyl siloxysilicate | 20.0 |
| (3) Compound 1 | 3.0 |
| (4) Candelilla wax | 8.0 |
| (5) Alkyl-modified silicone resin-coated black iron oxide | 10.0 |
| (6) 1,3-Butylene glycol | 3.0 |
| (7) Methyl paraben | 0.15 |
| (8) Ion-exchanged water | 20.0 |
| (9) Sodium 2-undecyl-N,N,N-(hydroxyethylcarboxymethyl) -2-imidazolate (Real content: 30%) | 4.0 |
| (10) Sodium chloride | 1.0 |

(Production method) (1) to (4) were heated to 80 ˚C, and (5) was mixed therein. To the obtained oil phase solution, an aqueous phase solution in which (7) to (10) were mixed and heated to 80 ˚C was gradually added. The mixture was stirred with a homomixer and cooled to obtain the W/O emulsion mascara of Formulation example 3.

## Claims

**1.** A complex consisting of:

an organosiloxane derivative represented by formula (1) or (2); and
an amphoteric surfactant and/or semipolar surfactant;

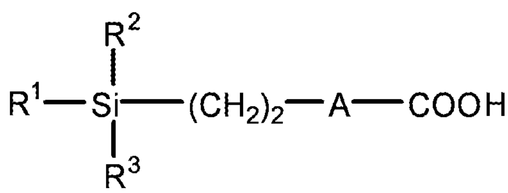

(1)

wherein at least one of $R^1$ to $R^3$ is a functional group represented by -O-Si$(R^4)_3$ in which $R^4$ is an alkyl group having 1 to 6 carbon atoms or a phenyl group, and remaining $R^1$ to $R^3$ may be same or different and each may be a substituted or unsubstituted monovalent hydrocarbon group; A is a linear or branched alkylene group represented by $C_qH_{2q}$ in which q is any integer of 0 to 20; and the organosiloxane derivative represented by the formula (1) contains a total of 2 to 100 silicon atoms (Si) on average per molecule; and

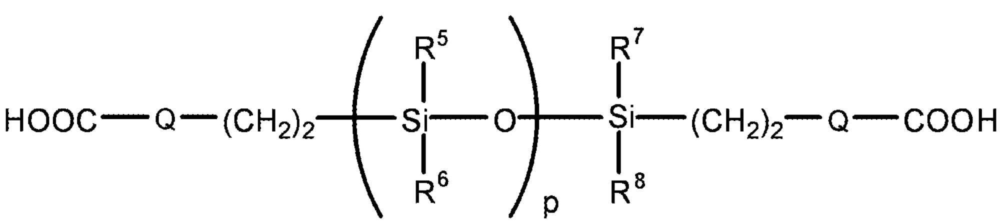

(2)

wherein $R^5$ to $R^8$ may be same or different and each is a substituted or unsubstituted monovalent hydrocarbon group; Q is a linear or branched alkylene group represented by $C_qH_{2q}$ in which q is any integer of 0 to 20; and p is any number of 0 to 100.

2. The complex according to claim 1 in which the organosiloxane derivative is represented by the formula (1), wherein $R^1$ and $R^2$ are respectively a functional group represented by $-O-Si(R^4)_3$ in which $R^4$ is an alkyl group having 1 to 6 carbon atoms; $R^3$ is a monovalent hydrocarbon group having 1 to 10 carbon atoms; and q is any integer of 6 to 20.

3. The complex according to claim 1 in which the organosiloxane derivative is represented by the formula (2), wherein $R^5$ to $R^8$ are respectively a group selected from group consisting of substituted or unsubstituted alkyl groups having 1 to 20 carbon atoms, aryl groups, and aralkyl groups; q is any integer of 6 to 20; and p is any number of 1 to 20.

4. The complex according to any of claims 1 to 3, wherein the amphoteric surfactant and/or semipolar surfactant is one or more selected from compounds represented by following formulas (A) to (F):

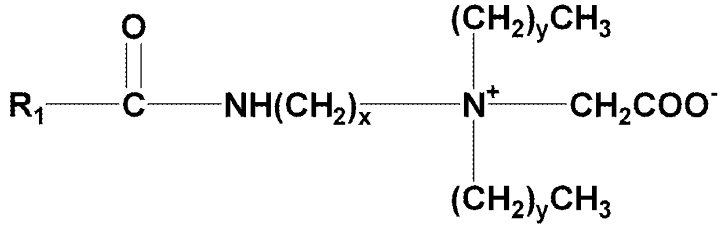

(A)

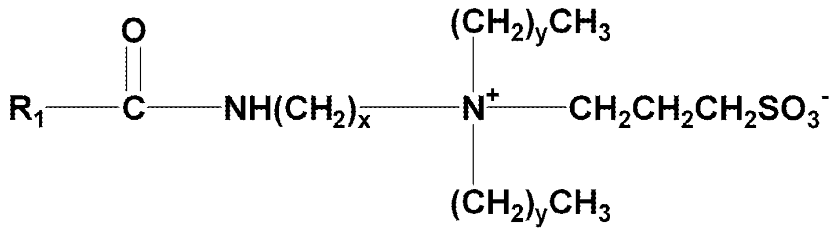

(B)

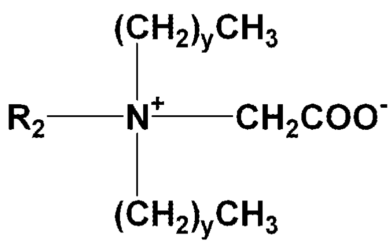

(C)

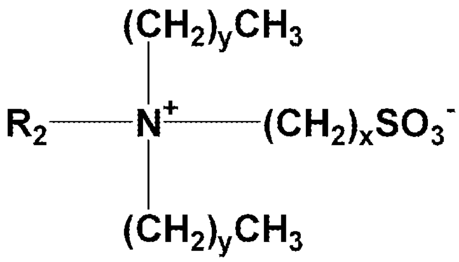

(D)

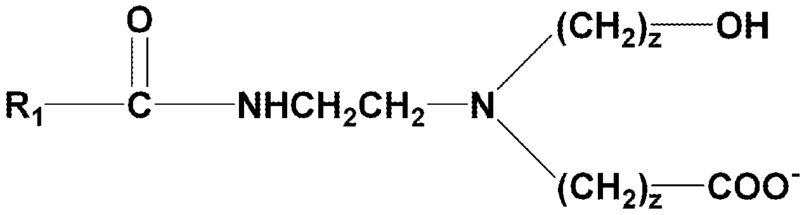

(E)

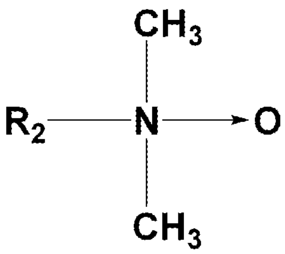

(F)

wherein $R_1$ is an alkyl group or an alkenyl group having 9 to 16 carbon atoms on average; $R_2$ is an alkyl group or an alkenyl group having 10 to 18 carbon atoms on average; x is any integer of 2 to 4; y is any integer of 0 to 4; and z is an integer of 1 or 2.

5. An emulsion containing the complex according to any of claims 1 to 4, an oil, and water.

6. A water-in-oil emulsion cosmetic containing the complex according to any of claims 1 to 4, an oil, and water, wherein a system is a water-in-oil type.

7. The water-in-oil emulsion cosmetic according to claim 6, further containing a salt and a wax ester, wherein the water-in-oil emulsion cosmetic is solid.

8. The solid water-in-oil emulsion cosmetic according to claim 7, containing a candelilla wax as the wax ester.

**INTERNATIONAL SEARCH REPORT**

International application No.
PCT/JP2009/063660

A. CLASSIFICATION OF SUBJECT MATTER
A61K8/891(2006.01)i, A61K8/06(2006.01)i, A61K8/40(2006.01)i, A61K8/92(2006.01)i, A61Q1/10(2006.01)i, B01F17/52(2006.01)i, C11D1/75(2006.01)i, C11D1/88(2006.01)i

According to International Patent Classification (IPC) or to both national classification and IPC

B. FIELDS SEARCHED

Minimum documentation searched (classification system followed by classification symbols)
A61K8/891, A61K8/06, A61K8/40, A61K8/92, A61Q1/10, B01F17/52, C11D1/75, C11D1/88

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P,A | JP 2008-266285 A (Shiseido Co., Ltd.), 06 November 2008 (06.11.2008), claims (Family: none) | 1-8 |
| E,A | JP 2009-185144 A (Shiseido Co., Ltd.), 20 August 2009 (20.08.2009), claims (Family: none) | 1-8 |
| E,A | WO 2009/22621 A1 (Shiseido Co., Ltd.), 19 February 2009 (19.02.2009), claims (Family: none) | 1-8 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
26 October, 2009 (26.10.09)

Date of mailing of the international search report
02 November, 2009 (02.11.09)

Name and mailing address of the ISA/
Japanese Patent Office

Authorized officer

Facsimile No.

Telephone No.

Form PCT/ISA/210 (second sheet) (April 2007)

**INTERNATIONAL SEARCH REPORT**

International application No.
PCT/JP2009/063660

C (Continuation). DOCUMENTS CONSIDERED TO BE RELEVANT

| Category* | Citation of document, with indication, where appropriate, of the relevant passages | Relevant to claim No. |
|---|---|---|
| E,A | WO 2009/25146 A1 (Shiseido Co., Ltd.), 26 February 2009 (26.02.2009), claims (Family: none) | 1-8 |
| A | JP 60-168532 A (Dow Corning Corp.), 02 September 1985 (02.09.1985), claims & US 4501619 A & EP 146756 A1 & DE 3471677 D & CA 1205350 A1 | 1-8 |
| A | JP 10-109926 A (BASF AG.), 28 April 1998 (28.04.1998), claims & US 6007801 A & EP 834300 A1 & DE 19639669 A & CA 2215131 A1 | 1-8 |
| A | JP 11-335563 A (Shin-Etsu Chemical Co., Ltd.), 07 December 1999 (07.12.1999), claims & US 6290942 B1 | 1-8 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008200660 A
- WO 9603107 A